# EUROPEAN PATENT SPECIFICATION

(11) **EP 0 527 037 B1**
(45) Date of publication and mention of the grant of the patent: **16.10.1996**
(21) Application number: 92307123.7
(22) Date of filing: 04.08.1992
(51) Int. Cl.: C07D 261/08, A01N 43/80

(54) **4-Benzoylisoxazole derivatives and their use as herbicides**
4-Benzoylisoxazol-Derivate und ihre Anwendung als Herbizide
Dérivés de benzoyl isoxazole et leur utilisation comme herbicides

(30) Priority: 05.08.1991 GB 9116833; 12.03.1992 US 850424
(43) Date of publication of application: 10.02.1993
(73) Proprietor: RHONE-POULENC AGRICULTURE LTD., Ongar,Essex CM5 0HW (GB)
(72) Inventor: Cain, Paul Alfred,c/o Research Station, Ongar,Essex CM5 0HW (GB); Cramp, Susan Mary,c/o Research Station, Ongar,Essex CM5 0HW (GB); Little, Gillian Mary,c/o Research Station, Ongar,Essex CM5 0HW (GB); Luscombe, Brian Malcolm,c/o Research Station, Ongar,Essex CM5 0HW (GB)
(74) Representative: Bentham, Stephen

(56) References cited:
- EP-A- 487 357
- EP-A- 0 418 175

## Description

This invention relates to novel 4-benzoyl isoxazole derivatives, compositions containing them and their use as herbicides.

EP-A-0,418,175 also discloses certain 4-benzoyl isoxazole derivatives useful as herbicides

EP-A-0,487,357, which was published after the two priority dates claimed for the present invention but before the date of filing and thus falls under Article 54(3) EPC, also discloses certain 4-benzoyl isoxazole derivatives useful as herbicides.

The present invention provides 4-benzoyl isoxazole derivatives of general formula (I): 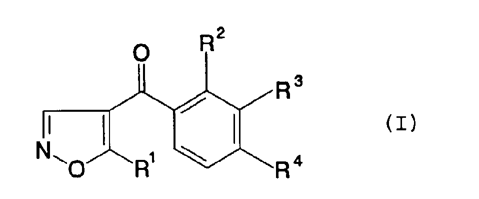 wherein:
R¹ represents:
   a methyl, ethyl, isopropyl, cyclopropyl or 1-methylcyclopropyl group;
R² represents:
   a straight- or branched-chain alkyl or alkoxy group containing up to four carbon atoms;
R³ represents:
   a hydrogen, chlorine, bromine or fluorine atom; or
   a group selected from R⁵, -CO₂R⁵ and -OR⁵; or
   a straight- or branched-chain alkyl group containing up to four carbon atoms substituted by -OR^{S}; or
   a straight- or branched-chain alkoxy group containing up to four carbon atoms substituted by -OR⁵;
R⁴ represents -S(O)ₙR;
R⁵ represents:
   a straight- or branched-chain alkyl group containing up to 4 carbon atoms which is optionally substituted by one or more halogen atoms;
   R represents a methyl or ethyl group; and
n represents zero, one or two;
   provided that when R¹ represents methyl or cyclopropyl, R² represents methyl and R⁴ represents -S0₂Me, R³ is a group other than -C0₂Me or -CO₂iPr;

which possess valuable herbicidal properties.

The compounds of the invention, in some aspects of their herbicidal activity, show advantages over known compounds.

A preferred class of compounds of formula (I) because of their herbicidal properties are those wherein;
a) R¹ represents:
   isopropyl, cyclopropyl or 1-methylcyclopropyl group; and/or
b) R² represents:
   a methyl, ethyl, methoxy or ethoxy group; and/or
c) R³ represents:
   a hydrogen, chlorine, bromine or fluorine atom; or
   a group selected from methyl, methoxy, ethoxy, -CH₂0R⁵,
   -O-(CH₂)₂OR⁵, and -C0₂R⁵, in which R⁵ represents a straight- or branched- chain alkyl group containing up to 3 carbon atoms.

A further preferred class of compounds of formula (I) are those wherein:-
R¹ represents isopropyl, cyclopropyl or 1-methylcyclopropyl;
R² represents methyl, ethyl, methoxy or ethoxy; and
R³ represents hydrogen, fluorine, chlorine, bromine, methyl, methoxymethyl or 1-methoxyethoxy

A further preferred class of compounds of formula (I) are those wherein:-
R¹ represents isopropyl, 1-methylcyclopropyl or cyclopropyl;
R² represents methyl, ethyl, methoxy or ethoxy;
R³ represents hydrogen, fluorine, chlorine, bromine, methyl or methoxy; and
R represents methyl.

A further preferred class of compounds of formula (I) are those wherein:-
R¹ represents cyclopropyl;
R² represents methyl or methoxy and R³ represents hydrogen or methoxy provided that R² and R³ do not simultaneously represent methoxy; and
R represents methyl.

Particularly preferred compounds because of their herbicidal properties include:-
A) 5-cyclopropyl-4-(2-methoxy-4-methylsulphenylbenzoyl)isoxazole;
B) 5-cyclopropyl-4-(2-methoxy-4-methylsulphonylbenzoyl)isoxazole;
C) 5-qyclopropyl-4-(2-methoxy-4-methylsulphinylbenzoyl)isoxazole;
D) 5-cyclopropyl-4-(2-methyl-4-methylsulphinylbenzoyl)isoxazole;
E) 5-cyclopropyl-4-(2-methyl-4-methylsulphonylbenzoyl)isoxazole;
F) 5-cyclopropyl-4-(2-methyl-4-methylsulphenylbenzoyl)isoxazole;
G) 5-(1 -methylcydopropyl)4-(2-methyl-4-methylsulphonylbenzoyl)-isoxazole;
H) 4-(2-ethyl-4-methylsulphenylbenzoyl)-5-cyclopropylisoxazole;
I) 4-(2-ethyl-4-methylsulphinylbenzoyl)-5-cyclopropylisoxazole;
J) 4-(2-ethyl-4-methylsulphonylbenzoyl)-5-cyclopropylisoxazole;
K) 4-(2-ethoxy-4-methylsulphenylbenzoyl)-5-cyclopropylisoxazole;
L) 4-(2-ethoxy-4-methylsulphonylbenzoyl)-5-cyclopropylisoxazole;
M) 4-(3-chloro-2-methyl-4-methylsulphenylbenzoyl)-5-cyclopropylisoxazole;
N) 4-(3-chloro-2-methyl-4-methylsulphinylbenzoyl)-5-cyclopropylisoxazole;
O) 4-(3-chloro-2-methyl-4-methylsulphonylbenzoyl)-5-cyclopropylisoxazole;
P) 4-(2-methyl-3-methoxy-4-methylsulphenylbenzoyl)-5-cyclopropylisoxazole;
Q) 4-(2-methyl-3-methoxy-4-methylsulphonylbenzoyl)-5-cyclopropylisoxazole;
R) 4-(2,3-dimethyl-4-methylsulphenylbenzoyl)-5-cyclopropylisoxazole,
S) 4-(2,3-dimethyl-4-methylsulphinylbenzoyl)-5-cyclopropylisoxazole and
T) 4-(2,3-dimethyl-4-methylsulphonylbenzoyl)-5-cyclopropylisoxazole.

The letters A to T are assigned to the above compounds for reference and identification hereafter

Compounds of general formula (l) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of general formula (I) may be prepared by the reaction of a compound of general formula (ll) : 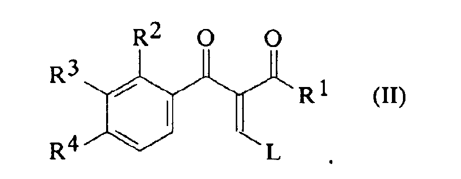 wherein L is a leaving group, with a salt of hydroxylamine. Hydroxylamine hydrochloride is generally preferred. Generally L is O-alkyl, for example ethoxy, or N,N-dialkylamino, for example dimethylamino. The reaction is generally carried out in a solvent such as ethanol or acetonitrile, optionally in the presence of a base or acid acceptor such as triethylamine or sodium acetate

According to a further feature of the present invention compounds of general formula (I) in which R⁴ represents a group -SR may be prepared by the reaction of a compound of general formula (lll): 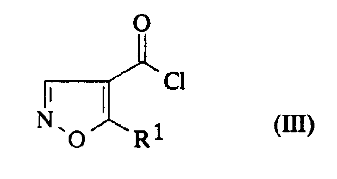 with a compound of general formula (lV): 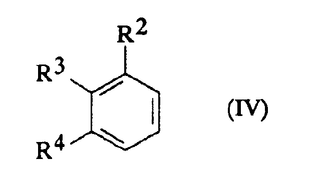 in which R⁴ represents a group -SR. The reaction is generally carried out in the presence of a Lewis acid catalyst such as aluminium chloride at a temperature between room temperature and 100°C.

According to a further feature of the present invention compounds of general formula (I) may be prepared by the reaction of a compound of general formula (V): 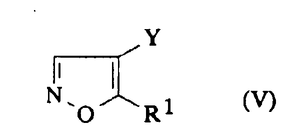 wherein Y represents a carboxy group or a reactive derivative thereof (such as a carboxylic acid chloride or carboxylic ester), or a cyano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

Intermediates in the preparation of compounds of general formula (I) are prepared by the application or adaptation of known methods.

Compounds of general formula (ll) may be prepared by the reaction of compounds of general formula (Vl): 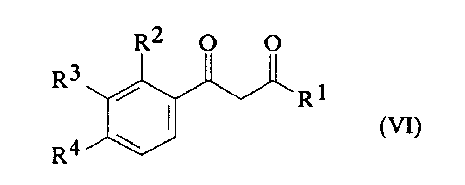 with either a trialkyl orthoformate or a dimethylformamide dialkyl acetal. Generally triethyl orthoformate or dimethylformamide dimethyl acetal are used. The reaction with a trialkyl orthoformate is generally carried out in the presence of acetic anhydride at the reflux temperature of the mixture and the reaction with a dimethylformamide dialkyl acetal is carried out optionally in the presence of an inert solvent at a temperature from room temperature to the reflux temperature of the mixture.

The preparation of compounds of general formulae (lll), (IV), (V) and (VI) is described extensively in the literature.

Those skilled in the art will appreciate that some compounds of general formula (I) may be prepared by the interconversion of other compounds of general formula (I) and such interconversions constitute yet more features of the present invention. Examples of such interconversions are hereafter described.

According to a further feature of the present invention compounds in which n is 1 or 2 may be prepared by the oxidation of the sulphur atom of compounds in which n is zero. The oxidation of the sulphur atom is generally carried out using for example 3-chloroperoxybenzoic acid in an inert solvent such as dichloromethane at a temperature from -40°C to room temperature, for example from -40°C to 0°C.

The following Examples illustrate the preparation of compounds of general formula (I) and the following Reference Examples illustrate the preparation of intermediates of the invention. In the present specification b.p means boiling point; m.p. means melting point. Where the letters NMR appear, the characteristics of the proton nuclear magnetic resonance spectrum follow

### EXAMPLE 1

### Compounds A, E, F, G, H, K, M, P, and R

Sodium acetate (7.87g) was added to a stirred mixture of 3-cyclopropyl -2-ethoxymethylene- 1-(2-methoxy-4-methylsulphenylphenyl) propan-1,3-dione (30.6g) and hydroxylamine hydrochloride (8.0g) in ethanol. The mixture was stirred at room temperature overnight, then evaporated to dryness and the residue was dissolved in ethyl acetate, washed with water, dried (anhydrous magnesium sulphate) and filtered. The filtrate was evaporated to dryness and the residue was triturated with ether and filtered to give 5-cyclopropyl-4-(2-methoxy-4-methylsulphenylbenzoyl)isoxazole as a white solid, m.p. 107.5- 108.5°C.

By proceeding in a similar manner the following compounds of general formula (I) were prepared from the appropriately substituted starting materials:

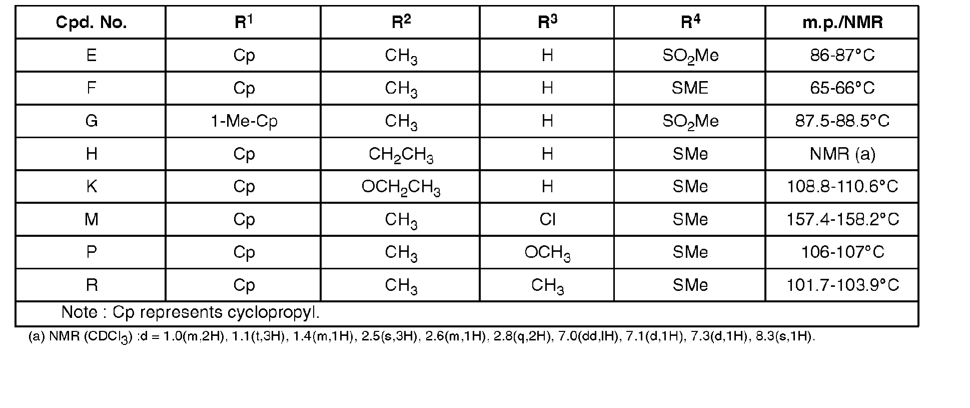

### EXAMPLE 2

### Compounds B, C, D, I, J, L, N, 0, Q, S and T.

3-Chloroperoxybenzoic acid (3.4g) was added to a solution of 5-qyclopropyl-4-(2-methoxy-4-methylsulphenylbenzoyl)isoxazole (3.5g) in dichloromethane at -15°C. The mixture was stirred at -15°C for 1 hour and at room temperature overnight. The mixture was recooled to -15°C and 3-chloro-peroxybenzoic acid (3.4g) was added. The mixture was filtered and the filtrate was washed with aqueous sodium metabisulphate solution, water, dried (anhydrous magnesium sulphate) and filtered. The filtrate was evaporated to dryness. The residue was purified by column chromatography on silica eluted with a mixture of ethyl acetate and hexane to give 5-cyclopropyl4-(2-methoxy-4-methylsulphonylbenzoyl) isoxazole (1.4g) as a white solid, m.p. 133-133.5°C and 5-cyclopropyl-4-(2-methoxy-4-methylsulphinylbenzoyl)isoxazole (0.85g) as an off-white solid, m.p. 100-102°C.

By proceeding in a similar manner the following compounds of general formula (I) were prepared from the appropriately substituted starting materials:

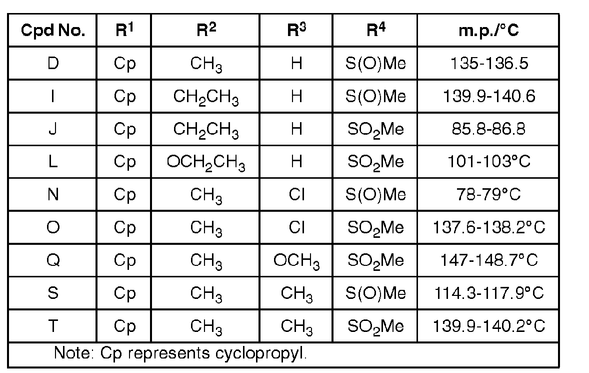

### REFERENCE EXAMPLE 1

A mixture of 3-cyclopropyl- 1-(2-methoxy-4-methylsulphenylphenyl)-propan-1,3-dione (25.4g) and triethyl orthoformate (39g) in acetic anhydride was stirred and heated at reflux for 3 hours. After cooling the mixture was evaporated to dryness and the residue was dissolved in toluene and re-evaporated to dryness to give 3-cyclopropyl-2-ethoxymethylene-1-(2-methoxy-4-methylsulphenylphenyl)-propan- 1,3-dione (30.6g) as a red gum which was not further purified.

By proceeding in a similar manner the following compounds were prepared from the appropriately substituted starting materials:

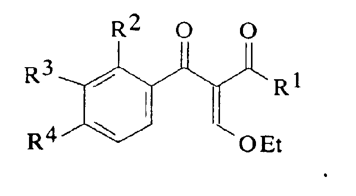

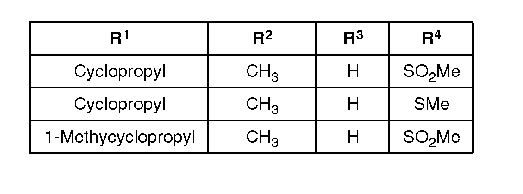

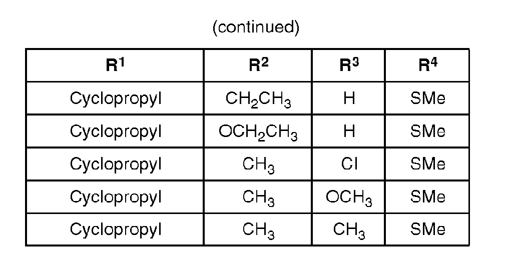

### REFERENCE EXAMPLE

A solution of t-butyl 3-cyclopropyl-2-(2-methoxy-4-methylsυlphenyl -benzoyl)-3-oxopropionate (35. lg) and 4-toluenesulphonic acid (1.2g) in toluene was stirred and heated at reflux for 8 hours. After cooling, it was washed with water, dried (anhydrous magnesium sulphate) and filtered. The filtrate was evaporated to dryness to give 3-cyclopropyl-1-(2-methoxy-4-methyl-sulphenylphenyl)-propan-1,3-dione (25.8g) as a red solid, NMR (CDCl₃); 0.7-1.2 (m,4H), 1.4-2.0 (m, 1H), 2.4(s,3H), 3.75 (s,3H), 6.4(s, 1H), 6.6(s, 1H), 6.65 (d, 1H) and 7.65 (d, 1H).

By proceeding in a similar manner the following compounds were prepared from the appropriately substituted starting materials:

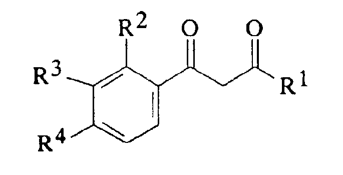

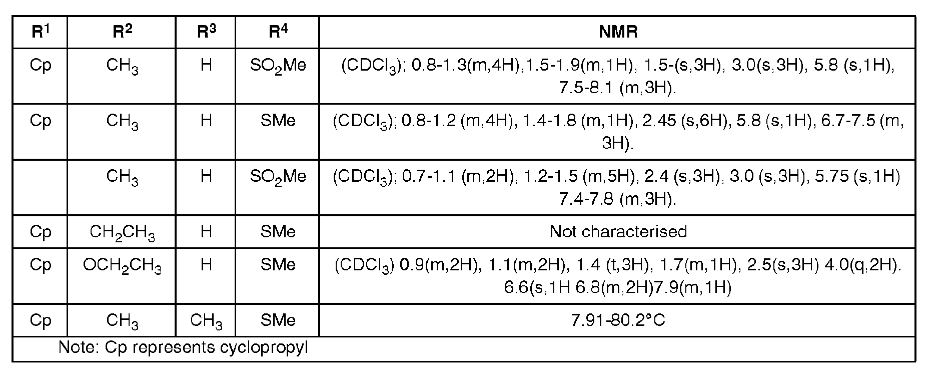

### REFERENCE EXAMPLE 3

Carbon tetrachloride (2ml) was added to a suspension of magnesium (2.67g) in methanol and the mixture was stirred and heated at reflux for 0.5 hours. After cooling t-butyl 3-cyclopropyl-3-oxopropionate (18.4g) was added. The mixture was stirred and heated at reflux for 0.5 hours. The mixture was evaporated to dryness and toluene was added to the residue. It was re-evaporated and the residue was suspended in acetonitrile. A solution of 2-methoxy-4-methylsulphenylbenzoyl chloride (21.8g) in acetonitrile was added and the mixture was stirred for 4 hours. It was evaporated to dryness and the residue was dissolved in ethyl acetate, washed with aqueous hydrochloric acid (2 M), water, dried (anhydrous magnesium sulphate) and filtered. The filtrate was evaporated to dryness to give t-butyl 3-cyclopropyl-2-(2-methoxy-4-methylsulphenylbenzoyl)-3-oxopropionate (36g) as a brown oil which was not purified further

By proceeding in a similar manner the following compounds were prepared from the appropriately substituted starting materials:

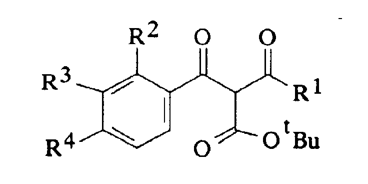

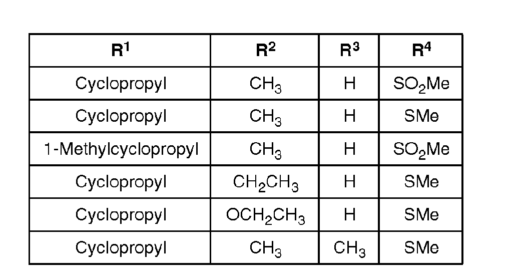

### REFERENCE EXAMPLE 4

A solution of cyclopropylmethyl ketone (6.5g) in THF was added to a stirred suspension of sodium hydride (2.88g) in THF under an inert atmosphere and the mixture was stirred for 3.5 hours. After this time a solution of ethyl 3-chloro-2-methyl-4-(methylsulphenyl)benzoate (8.5g) in THF was added. The resultant mixture was stirred overnight at room temperature. After this time the reaction mixture was diluted with ether and 2M hydrochloric acid. The layers were separated and the organic layer was washed with water, dried (MgS0₄) and filtered. The filtrate was evaporated to dryness to give a crude yellow solid. This solid was purified by column chromatography on silica eluted with a mixture of ethyl acetate and petrol (b.p.60-80°C) yielding 3-cyclopropyl-1-(3-chloro-2-methyl-4-methylsulphenylphenyl)-pro- pan-1,3-dione (5.25g) as a yellow solid, m.p.113-116°C.

By proceeding in a similar manner the following compound was prepared from the appropriately substituted starting material:
3-cyclopropyl-1-(2-methyl-3-methoxy-4-methylsulphenylphenyl)propan-1,3-dione; NMR (CDCl₃); 0.8-1.2(m,4H), 1.6(m,1H), 2.3(s,3H), 2.4(s,3H), 3.7(s,3H), 5.8(s, 1 H); 6.8(d, 1H), 7.2(d, 1H), 16.0(bs, 1H).

Benzoyl chlorides were prepared by heating the appropriately substituted benzoic acids at reflux with thionyl chloride for 3 hours. The excess thionyl chloride was removed by evaporation and the benzoyl chlorides were used directly without further purification.

According to a feature of the present invention, there is provided a method for controlling the growth of weeds (i. e. undesired vegetation) at a locus which comprises applying to the locus a herbicidally effective amount of at least one isoxazole derivative of general formula (I). For this purpose, the isoxazole derivatives are normally used in the form of herbicidal compositions (i.e. in association with compatible diluents or carriers and/or surface active agents suitable for use in herbicidal compositions), for example as hereinafter described

The compounds of general formula (I) show herbicidal activity against dicotyledonous (i.e. broad-leafed) and mono- cotyledonous (i.e. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:
broad-leafed weeds, for example, Abutilon theophrasti, Amaranthus retroflexus, Bidens pilosa, Chenopodium album, Galium aparine, Ipomoea spp. eg lpomoea purpurea, Sesbania exalta, Sinapis arvensis, Solanum nigrum and Xanthium strumarium, and
grass weeds, for example Alopecurus myosuroides, Avena fatua, Diqitaria sanquinalis,Echinichloa crus-galli, Sorghum bicolor, Eleusine indica and Setaria spp, e.g. Setaria faberii or Setaria viridis, and
sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a cropgrowing area, the rate of application should be sufficient to control the growth of weeds without causing substantial permanent damage to the crop. In general, taking these factors into account, application rates between 0.01 kg and 5kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e. g. the crops hereinbefore mentioned, application rates between 0.001 kg and 4.0kg, and preferably between 0.001 kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not cropgrowing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop- growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 1 O.Okg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a preemergence control on later-germinating weeds in the soil

Where especially prolonged weed control is required, the application of the compounds of general formula (I) may be repeated if required.

According to a further feature of the present invention, there are provided compositions suitable for herbicidal use comprising one or more of the isoxazole derivatives of general formula (I), in association with, and preferably homogeneously dispersed in, one or more compatible agriculturally-acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
aqueous suspension concentrates which comprise from 10 to 70% of one or more compounds of general formula (I), from 2 to 10% of surface-active agent, from 0.1 to 5% of thickener and from 15 to 87.9% of water;
wettable powders which comprise from 10 to 90% of one or more compounds of general formula (1), from 2 to 10% of surface-active agent and from 8 to 88% of solid diluent or carrier;
water soluble or water dispersible powders which comprise from 10 to 90% of one or more compounds of general formula (1), from 2 to 40% of sodium carbonate and from 0 to 88% of solid diluent;
liquid water soluble concentrates which comprise from 5 to 50%, e.g. 10 to 30%, of one or more compounds of general formula (I), from 5 to 25% of surface-active agent and from 25 to 90%, e.g. 45 to 85 %, of water miscible solvent, e.g. dimethylformamide, or a mixture of water-miscible solvent and water;
liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;
granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6,-diethyl-N-(methoxy-methyl)-acetanilide], atrazine [2-chloro-4-ethylamino-6 -isopropyiamino-1,3,5-triazine], bromoxynil [3,5-dibromo-4hydroxybenzonitrile], chlortoluron [N'-(3-chloro-4-methylphenyl)-N,N-dimethylurea], cyanazine [2-chloro-4-(1-cyano-1- methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2- dimethyl-3,5-diphenylpyrazolium salts], flampropmethyl [methyl N-2-(N-benzoyl-3-chloro-4-fluoroanilino)-propionate], fluometuron [N'-(3-trifluoro- methylphenyl)-N,N-dimethylurea], isoproturon [N' -(4-isopropylphenyl)-N,N-dimethylυrea], insecticides, e.g. synthetic pyrethroids, e.g. permethrin and cyper- methrin, and fungicides, e.g. carbamates, e.g. methyl N-(1-butyl-carbamoyl-benzimidazol-2-yl)carbamate, and triazoles e.g. 1-(4-chloro-phenoxy)-3,3- dimethyl- 1-( 1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula (I) or, as is preferred, a herbicidal composition as hereinbefore described, and preferably a herbicidal concentrate which must be diluted before use, comprising at least one of the isoxazole derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling The instructions will be physically associated with the container, for example by being printed directly thereon or on a label or tag affixed thereto. The directions will normally indicate that the contents of the container, after dilution if necessary, are to be applied to control the growth of weeds at rates of application between 0.01 kg and 20kg of active material per hectare in the manner and for the purposes hereinbefore described.

The following Examples illustrate herbicidal compositions according to the present invention:

### EXAMPLE C1

A soluble concentrate is formed from :

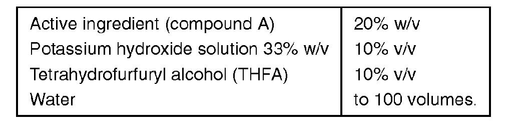

by stirring THFA, active ingredient (compound A) and 90% volume of water and slowly adding the potassium hydroxide solution until a steady pH 7-8 is obtained then making up to volume with water.

Similar soluble concentrates may be prepared as described above by replacing the isoxazole (compound A) with other compounds of general formula (I).

### EXAMPLE C2

A wettable powder is formed from :

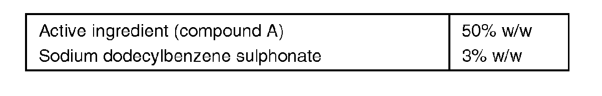

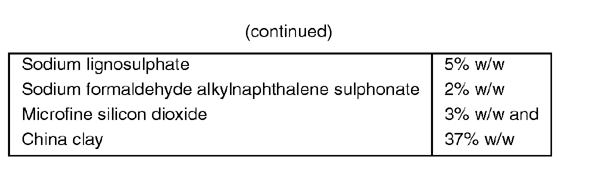

by blending the above ingredients together and grinding the mixture in an air jet mill.

Similar wettable powders may be prepared as described above by replacing the isoxazole (compound A) with other compounds of general formula (I).

### EXAMPLE C3

A water soluble powder is formed from :

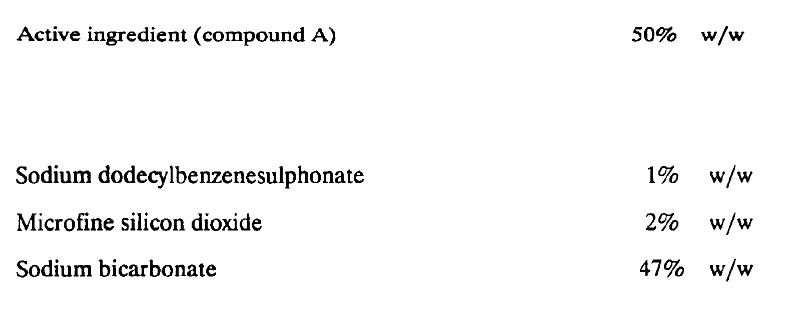

by mixing the above ingredients and grinding the above mixture in a hammer mill.

Similar water soluble powders may be prepared as described above by replacing the isoxazole (compound A) with other compounds of general formula (I).

The compounds of the invention have been used in herbicidal applications according to the following procedures.

### METHOD OF USE OF HERBICIDAL COMPOUNDS;

### a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare

### b) Weed control : Pre-emerqence

The seeds were sown in 70 mm square, 75 mm deep plastic pots in non-sterile soil . The quantities of seed per pot were as follows:-

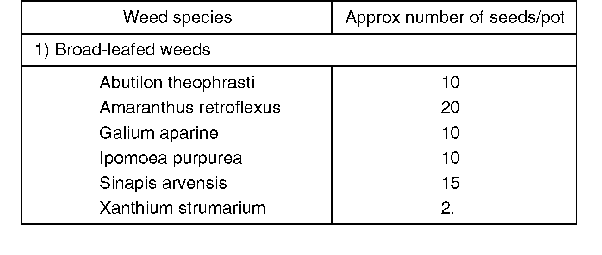

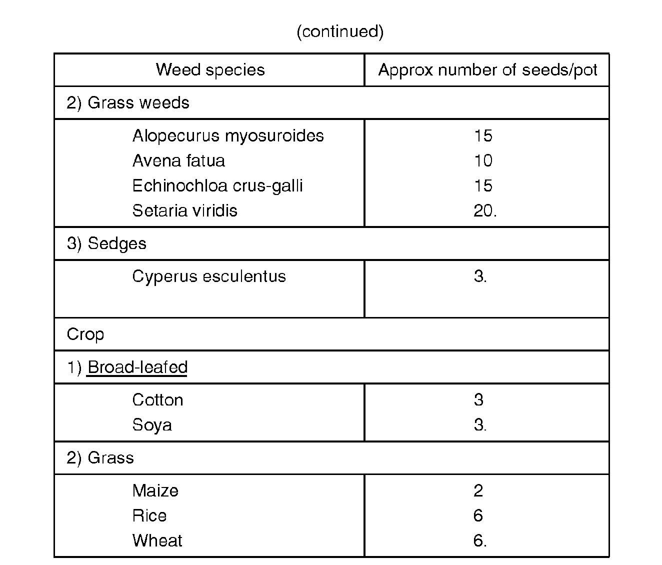

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead Visual assessment of crop damage was made 2024 days after spraying. The results were expressed as the percentage reduction in growth or damage to the crop or weeds, in comparison with the plants in the control pots.

### c) Weed control : Post-emer^{q}ence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-
1) Broad leafed weeds 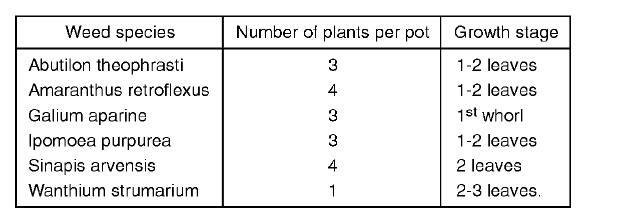
2) Grass weeds 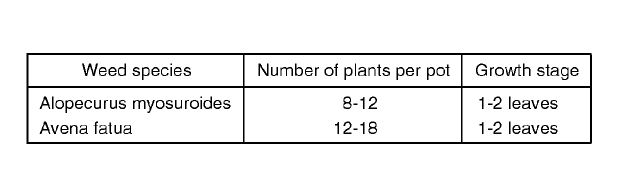 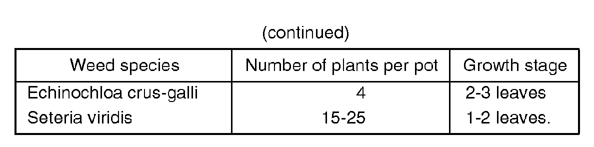
3) Sedges 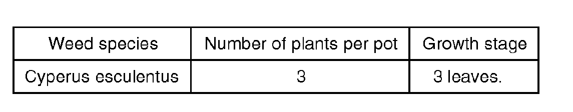
1) Broad leafed 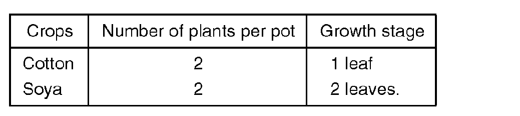
2) Grass

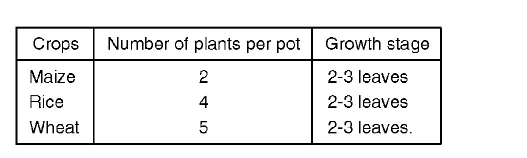

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled subirrigation. Visual assessment of crop damage and weed control was made 20-24 days after spraying. The results were expressed as the percentage reduction in growth or damage to the crop or weeds, in comparison with the plants in the control pots.

The compounds of the invention, used at 4kg/ha or less,have shown an excellent level of herbicidal activity together with crop tolerance on the weeds used in the foregoing experiments.

When applied pre- or post-emergence at 1000g/ha compounds A to T gave at least 90% reduction in growth of one or more of the weed species.

## Claims (Claims for the following Contracting State(s) : AT, BE, CH, LI, DE, DK, FR, GB, GR, IT, LU, NL, PT, SE, IE)

1. 4-Benzoyl isoxazole derivatives of general formula (I): 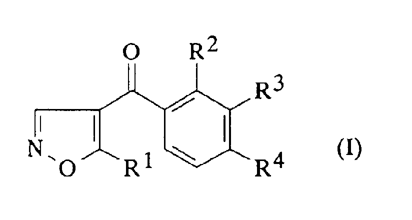 wherein:
R¹ represents:
a methyl, ethyl, isopropyl, cyclopropyl or 1-methylcyclopropyl group;
R² represents:
a straight- or branched- chain alkyl or alkoxy group containing up to four carbon atoms;
R³ represents:
a hydrogen, chlorine, bromine or fluorine atom; or
a group selected from R⁵,-C02R⁵ and -OR⁵; or
a straight- or branched- chain alkyl group containing up to four carbon atoms substituted by -OR⁵; or
a straight- or branched- chain alkoxy group containing up to four carbon atoms substituted by -OR⁵;
R⁴ represents -S(O)ₙR;
R⁵ represents:
a straight- or branched- chain alkyl group containing up to 4 carbon atoms which is optionally substituted by one or more halogen atoms;
R represents a methyl or ethyl group; and
n represents zero, one or two;
provided that when R⁷ represents methyl or cyclopropyl, R² represents methyl and R⁴ represents -S0₂Me, R³ is a group other than -C0₂Me or -C0₂iPr.

2. A compound according to claim 1 wherein:
a) R represents: isopropyl, cyclopropyl or 1-methylcyclopropyl group; and/or
b) R² represents:
a methyl, ethyl, methoxy or ethoxy group; and/or
c) R³ represents:
a hydrogen chlorine, bromine or fluorine atom; or
a group selected from methyl, methoxy, ethoxy, -CH₂0R⁵,
-O-(CH₂)₂OR⁵, and -CO₂R⁵; in which R⁵ represents a straight- or branched- chain alkyl group containing up to 3 carbon atoms

3. A compound according to claim 1 or 2 wherein:-
R¹ represents isopropyl, cyclopropyl or 1-methylcyclopropyl;
R² represents methyl, ethyl, methoxy or ethoxy; and
R³ represents hydrogen, fluorine, chlorine, bromine, methyl, methoxymethyl or 1-methoxyethoxy

4. A compound according to claim 1, 2 or 3 wherein:-
R¹ represents isopropyl, 1-methylcyclopropyl or cyclopropyl;
R² represents methyl, ethyl, methoxy or ethoxy;
R³ represents hydrogen, fluorine, chlorine, bromine, methyl or methoxy; and
R represents methyl.

5. A compound according to any one of the preceding claims wherein:-
R¹ represents cyclopropyl;
R² represents methyl or methoxy and R³ represents methoxy or hydrogen provided that R² and R³ do not simultaneously represent methoxy;
and R represents methyl.

6. A compound according to claim 1 which is
5-cyclopropyl-4-(2-methoxy-4-methylsulphenylbenzoyl)isoxazole;
5-cyclopropyl-4-(2-methoxy-4-methylsulphonylbenzoyl)isoxazole;
5-cyclopropyl-4-(2-methoxy-4-methylsulphinylbenzoyl)isoxazole;
5-cyclopropyl-4-(2-methyl-4-methylsulphinylbenzoyl)isoxazole;
5-cyclopropyl-4-(2-methyl-4-methylsulphonylbenzoyl)isoxazole;
5-cyclopropyl-4-(2-methyl-4-methylsulphenylbenzoyl)isoxazole;
5-(1-methylcyclopropyl)-4-(2-methyl-4-methylsulphonylbenzoyl)-isoxazole;
4-(2-ethyl-4-methylsulphenylbenzoyl)-5-cyclopropylisoxazole;
4-(2-ethyl-4-methylsulphinylbenzoyl)-5-cyclopropylisoxazole;
4-(2-ethyl-4-methylsulphonylbenzoyl)-5-cyclopropylisoxazole;
4-(2-ethoxy-4-methylsulphenylbenzoyl)-5-cyclopropylisoxazole;
4-(2-ethoxy-4-methylsulphonylbenzoyl)-5-cyclopropylisoxazole;
4-(3-chloro-2-methyl-4-methylsulphenylbenzoyl)-5-cyclopropylisoxazole;
4-(3-chloro-2-methyl4-methylsulphinylbenzoyl)-5-cyclopropylisoxazole;
4-(3-chloro-2-methyl-4-methylsulphonylbenzoyl)-5-cyclopropylisoxazole;
4-(2-methyl-3-methoxy-4-methylsulphenylbenzoyl)-5-cyclopropylisoxazole;
4-(2-methyl-3-methoxy-4-methylsulphonylbenzoyl)-5-cyclopropylisoxazole ;
4-(2,3-dimethyl-4-methylsulphenylbenzoyl)-5-cyclopropylisoxazole;
4-(2,3-dimethyl-4-methylsulphinylbenzoyl)-5-cyclopropylisoxazole or
4-(2,3-dimethyl-4-methylsulphonylbenzoyl)-5-cyclopropylisoxazole.

7. A process for the preparation of a compound of general formula (I) according to claim 1 which comprises:
a) the reaction of a compound of general formula (ll): 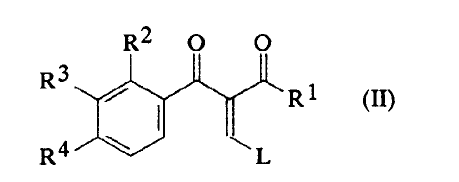 wherein L is a leaving group and the other symbols are as defined in claim 1, with a salt of hydroxylamine,
b) where R⁴ represents a group -SR, the reaction of a compound of general formula (lll): 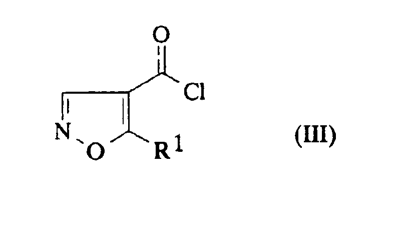 wherein R' is as defined in claim 1, with a compound of general formula (lV): 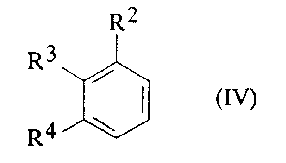 in which R⁴ represents a group -SR and R, R² and R³ are as defined in claim 1;
c) the reaction of a compound of general formula (V): 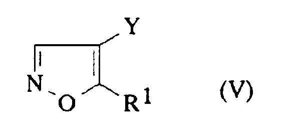 wherein Y represents a carboxy group or a reactive derivative thereof, or a cyano group and R¹ is as defined in claim 1, with an appropriate organometallic reagent;
d) where n represents 1 or 2, the oxidation of the sulphur atom of the corresponding compound of general formula (I) in which n is zero.

8. A herbicidal composition which comprises as active ingredient a herbicidally effective amount of an isoxazole derivative of general formula (I) as defined in claim 1 in association with an agriculturally acceptable diluent or carrier and/or surface active agent.

9. A herbicidal composition according to claim 8 which comprises 0.05 to 90% by weight of active ingredient.

10. A herbicidal composition according to claim 8 or 9 which is in liquid form and contains from 0.05 to 25% of surface-active agent.

11. A herbicidal composition according to claim 8, 9 or 10 in the form of an aqueous suspension concentrate, a wettable powder, a water soluble or water dispersible powder, a liquid water soluble concentrate, a liquid emulsifiable suspension concentrate, a granule or an emulsifiable concentrate.

12. A method for controlling the growth of weeds at a locus which comprises applying to the locus a herbicidally effective amount of an isoxazole derivative of general formula (I) as defined in claim 1.

13. A method according to claim 12 in which the locus is an area used, or to be used, for growing of crops and the compound is applied at an application rate from 0.01 kg to 4.0 kg per hectare.

14. A method according to claim 12 in which the locus is an area which is not a crop-growing area and the compound is applied at an application rate from 1.0 kg to 20.0 kg per hectare.

## Claims (Claims for the following Contracting State(s) : ES)

1. A process for the preparation of a 4-benzoyl isoxazole derivative of general formula (I): 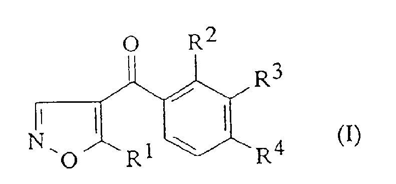 wherein:
R¹ represents:
a methyl, ethyl, isopropyl, cyclopropyl or 1-methylcyclopropyl group;
R² represents:
a straight- or branched- chain alkyl or alkoxy group containing up to four carbon atoms;
R³ represents:
a hydrogen, chlorine, bromine or fluorine atom; or
a group selected from R⁵,-CO₂R⁵ and -OR⁵; or
a straight- or branched- chain alkyl group containing up to four carbon atoms substituted by -OR⁵; or
a straight- or branched- chain alkoxy group containing up to four carbon atoms substituted by -OR⁵;
R⁴ represents -S(O)ₙR;
R⁵ represents:
a straight- or branched- chain alkyl group containing up to 4 carbon atoms which is optionally substituted
by one or more halogen atoms;
R represents a methyl or ethyl group; and
n represents zero, one or two;
provided that when R¹ represents methyl or cyclopropyl, R² represents methyl and R⁴ represents -SO₂Me, R³ is a group other than -C0₂Me or -C0₂iPr;
which process comprises:
a) the reaction of a compound of general formula (ll): 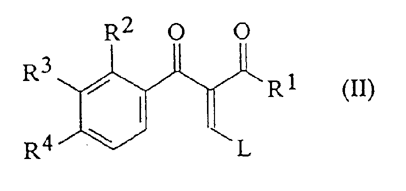 wherein L is a leaving group and the other symbols are as hereinbefore defined, with a salt of hydroxylamine;
b) where R⁴ represents a group -SR, the reaction of a compound of general formula (lll): 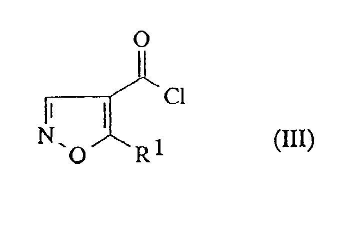 wherein R¹ is as hereinbefore defined, with a compound of general formula (IV): 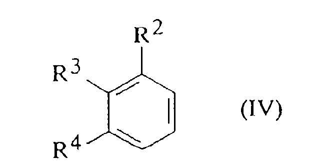 in which R⁴ represents a group -SR and R, R² and R³ are as hereinbefore defined;
c) the reaction of a compound of general formula (V): 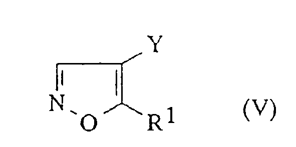 wherein Y represents a carboxy group or a reactive derivative thereof, or a cyano group and R¹ is as hereinbefore defined, with an appropriate organometallic reagent; or
d) where n represents 1 or 2, the oxidation of the sulphur atom of the corresponding compound of general formula (I) in which n is zero.

2. A process according to claim 1 wherein:
a) R¹ represents: isopropyl, cyclopropyl or 1-methylcyclopropyl group; and/or
b) R² represents:
a methyl, ethyl, methoxy or ethoxy group; and/or
c) R³ represents:
a hydrogen, chlorine, bromine or fluorine atom or
a group selected from methyl, methoxy, ethoxy, -CH₂0R⁵,
-O-(CH₂)₂OR⁵, and -CO₂R⁵_{;} in which R⁵ represents a straight- or branched- chain alkyl group containing up to 3 carbon atoms.

3. A process according to claim 1 or 2 wherein:-
R¹ represents isopropyl, cyclopropyl or 1-methylcyclopropyl;
R² represents methyl, ethyl, methoxy or ethoxy; and
R³ represents hydrogen, fluorine, chlorine, bromine, methyl, methoxymethyl or 1-methoxyethoxy

4. A process according to claim 1, 2 or 3 wherein:-
R¹ represents isopropyl, 1-methylcyclopropyl or cyclopropyl;
R² represents methyl, ethyl, methoxy or ethoxy;
R³ represents hydrogen, fluorine, chlorine, bromine, methyl or methoxy; and
R represents methyl.

5. A process according to any one of the preceding claims wherein:-
R¹ represents cyclopropyl;
R² represents methyl or methoxy and R³ represents methoxy or hydrogen provided that R² and R³ do not simultaneously represent methoxy,
and R represents methyl

6. A process according to claim 1 for the preparation of:
5-cyclopropyl-4-(2-methoxy-4-methylsulphenylbenzoyl)isoxazole;
5 -cyclopropyl-4-(2-methoxy-4-methylsulphonylbenzoyl)isoxazole;
5-cyclopropyl-4-(2-methoxy-4-methylsulphinylbenzoyl)isoxazole;
5-cyclopropyl-4-(2-methyl-4-methylsulphinylbenzoyl)isoxazole;
5-cyclopropyl-4-(2-methyl-4-methylsulphonylbenzoyl)isoxazole;
5-cyclopropyl-4-(2-methyl-4-methylsulphenylbenzoyl)isoxazole;
5-(1-methylcyclopropyl)-4-(2-methyl-4-methylsulphonylbenzoyl)-isoxazole;
4-(2-ethyl-4-methylsulphenylbenzoyl)-5-cyclopropylisoxazole;
4-(2-ethyl-4-methylsulphinylbenzoyl)-5-cyclopropylisoxazole;
4-(2-ethyl-4-methylsulphonylbenzoyl)-5-cyclopropylisoxazole;
4-(2-ethoxy-4-methylsulphenylbenzoyl)-5-cyclopropylisoxazole;
4-(2-ethoxy4-methylsulphonylbenzoyl)-5-cyclopropylisoxazole;
4-(3-chloro-2-methyl-4-methylsulphenylbenzoyl)-5-cyclopropylisoxazole;
4-(3-chloro-2-methyl-4-methylsulphinylbenzoyl)-5-cyclopropylisoxazole;
4-(3-chloro-2-methyl-4-methylsulphonylbenzoyl)-5-cyclopropylisoxazole;
4-(2-methyl-3-methoxy-4-methylsulphenylbenzoyl)-5-cyclopropylisoxazole;
4-(2-methyl-3-methoxy-4-methylsulphonylbenzoyl)-5-cyclopropylisoxazole;
4-(2,3-dimethyl-4-methylsulphenylbenzoyl)-5-cyclopropylisoxazole;
4-(2,3-dimethyl-4-methylsulphinylbenzoyl)-5-cyclopropylisoxazole or
4-(2,3-dimethyl-4-methylsulphonylbenzoyl)-5-cyclopropylisoxazole.

7. A process for the preparation of a herbicidal composition which comprises formulating as active ingredient a herbicidally effective amount of an isoxazole derivative of general formula (I) as defined in claim 1 in association with an agriculturally acceptable diluent or carrier and/or surface active agent.

8. A process according to claim 7 for the preparation of a herbicidal composition which comprises 0.05 to 90% by weight of active ingredient.

9. A process according to claim 7 or 8 for the preparation of a herbicidal composition which is in liquid form and contains from 0.05 to 25% of surface-active agent.

10. A process according to claim 9, 10 or 11 for the preparation of a herbicidal composition in the form of an aqueous suspension concentrate, a wettable powder, a water soluble or water dispersible powder, a liquid water soluble concentrate, a liquid emulsifiable suspension concentrate, a granule or an emulsifiable concentrate.

11. A method for controlling the growth of weeds at a locus which comprises applying to the locus a herbicidally effective amount of an isoxazole derivative of general formula (I) as defined in claim 1.

12. A method according to claim 11 in which the locus is an area used, or to be used, for growing of crops and the compound is applied at an application rate from 0.01 kg to 4.0 kg per hectare.

13. A method according to claim 11 in which the locus is an area which is not a crop-growing area and the compound is applied at an application rate from 1.0 kg to 20.0 kg per hectare.

## Patentansprüche (Patentansprüche für folgende(n) Vertragsstaat(en) : AT, BE, CH, DE, DK, FR, GB, GR, IE, IT, LI, LU, NL, PT, SE)

1. 4-Benzoylisoxazolderivate der allgemeinen Formel (l) : 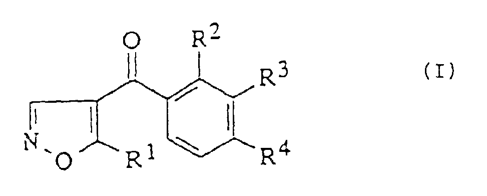 worin bedeuten:
R¹: Methyl, Ethyl, Isopropyl, Cyclopropyl oder 1-Methylcyclopropyl;
R²: eine geradkettige oder verzweigte Alkyl- oder Alkoxygruppe mit bis zu vier Kohlenstoffatomen;
R³: ein Wasserstoffatom, Chloratom, Bromatom oder Fluoratom; oder
eine unter R⁵,-C0₂R⁵ und -OR⁵ ausgewählte Gruppe; oder
eine geradkettige oder verzweigte Alkylgruppe mit bis zu vier Kohlenstoffatomen, die mit -OR⁵ subsituiert ist; oder
eine geradkettige oder verzweigte Alkoxygruppe mit bis zu vier Kohlenstoffatomen, die mit-OR⁵ substituiert ist;
R4: -S(O)ₙ^{R};
R⁵: eine geradkettige oder verzweigte Alkylgruppe mit bis zu vier Kohlenstoffatomen, die ggf. mit einem oder mehreren Halogenatomen substituiert ist;
R Methyl oder Ethyl; und
n: Null, eins oder zwei;
mit der Maßgabe, daß falls R¹ Methyl oder Cyclopropyl, R² Methyl und R⁴ -S0₂Me bedeuten, R³ keine -C0₂Me oder -CO₂iPr-Gruppe ist.

2. Verbindung nach Anspruch 1, worin bedeuten:
a) R¹: Isopropyl, Cyclopropyl oder l-Methylcyclopropyl; und/oder
b) R²: Methyl, Ethyl, Methoxy oder Ethoxy; und/oder
c) R³: ein Wasserstoffatom, Chloratom, Bromatom oder Fluoratom; oder
eine Gruppe, die unter Methyl, Methoxy, Ethoxy, -CH₂0R⁵, -O-(CH₂)₂OR⁵, und -CO₂R⁵ ausgewählt ist, worin R⁵ eine geradkettige oder verzweigte Alkylgruppe mit bis zu drei Kohlenstoffatomen bedeutet.

3. Verbindung nach Anspruch 1 oder 2, worin bedeuten:
R¹: Isopropyl, Cyclopropyl oder 1-Methylcyclopropyl;
R²: Methyl, Ethyl, Methoxy oder Ethoxy, und
R³ : Wasserstoff, Fluor, Chlor, Brom, Methyl, Methoxymethyl oder 1-Methoxyethoxy.

4. Verbindung nach Anspruch 1, 2 oder 3, worin bedeuten:
R¹: Isopropyl, 1-Methylcyclopropyl oder Cyclopropyl;
R²: Methyl, Ethyl, Methoxy oder Ethoxy;
R³ : Wasserstoff, Fluor, Chlor; Brom, Methyl oder Methoxy; und
R: Methyl.

5. Verbindung nach einem der vorhergehenden Ansprüche, worin bedeuten:
R¹: Cyclopropyl;
R²: Methyl oder Methoxy und R³: Methoxy oder Wasserstoff, mit der Maßgabe, daß R² und R³ nicht gleichzeitig Methoxy bedeuten;
und
R Methyl.

6. Verbindung nach Anspruch 1, die ist;
5-Cyclopropyl-4-(2-methoxy-4-methylsulphenylbenzoyl) isoxazol;
5-Cyclopropyl-4-(2-methoxy-4-methylsulphonylbenzoyl) isoxaol;
5-Cyclopropyl-4-(2-methoxy-4-methylsulphinylbenzoyl) isoxazol;
5-cyclopropyl-4-(2-methyl-4-methylsulphinylbenzoyl) isoxazol;
5-Cyclopropyl-4-(2-methyl-4-methylsulphonylbenzoyl) isoxazol;
5-Cyclopropyl-4-(2-methyl-4-methylsulphenylbenzoyl) isoxazol;
5-(1-Methylcyclopropyl)-4-(2-methyl-4-methylsulphonylbenzoyl)-isoxazol;
4-(2-Ethyl-4-methylsulphenylbenzoyl)-5-cyclopropylisoxazol;
4-(2-Ethyl-4-methylsulphinylbenzoyl)-5-cyclopropylisoxazol;
4-(2-Ethyl-4-methylsulphonylbenzoyl)-5-cyclopropylisoxazol;
4-(2-Ethoxy-4-methylsulphenylbenzoyl)-5-cyclopropylisoxazol;
4-(2-Ethoxy-4-methylsulphonylbenzoyl)-5-cyclopropylisoxazol;
4-(3-Chlor-2-methyl-4-methylsulphenylbenzoyl)-5-cyclopropylisoxazol;
4-(3-Chlor-2-methyl-4-methylsulphinylbenzoyl)-5-cyclopropylisoxazol;
4-(3-Chlor-2-methyl-4-methylsulphonylbenzoyl)-5-cyclopropylisoxazol;
4-(2-Methyl-3-methoxy-4-methylsulphenylbenzoyl)-5-cyclopropylisoxazol;
4-(2-Methyl-3-methoxy-4-methylsulphonylbenzoyl)-5-cyclopropylisoxazol;
4-(2,3-Dimethyl-4-methylsulphenylbenzoyl)-5-cyclopropylisoxazol;
4-(2,3-Dimethyl-4-methylsulphinylbenzoyl)-5-cyclopropylisoxazol; oder
4-(2,3-Dimethyl-4-methylsulphonylbenzoyl)-5-cyclopropylisoxazol.

7. Verfahren zur Herstellung einer Verbindung der allgemeinen Formel (I) nach Anspruch 1, das umfaßt:
a) Umsetzung einer Verbindung der allgemeinen Formel (II): 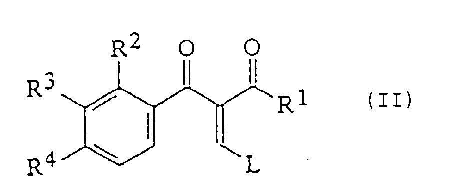 worin L eine austretende Gruppe bedeutet, und die anderen Symbole so sind, wie sie in Anspruch 1 definiert wurden,
mit einem Hydroxylaminsalz;
b) falls R⁴ eine Gruppe -SR bedeutet, Umsetzung einer Verbindung der allgemeinen Formel (lll) : 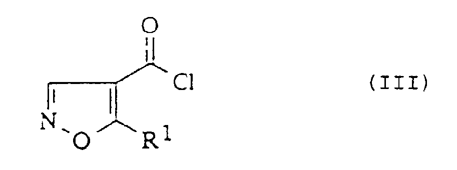 mit R¹ wie in Anspruch definiert,
mit einer Verbindung der allgemeinen Formel (IV) : 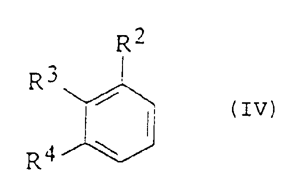 in der R⁴ eine Gruppe -SR bedeutet, und R, R² und R³ wie in Anspruch 1 definiert vorliegen;
c) Umsetzung einer Verbindung der allgemeinen Formel (V) : 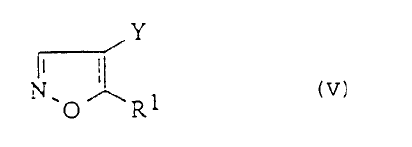 worin Y eine Carboxygruppe oder ein reaktives Derivat einer Carboxygruppe oder eine Cyanogruppe bedeutet, und R¹ wie in Anspruch 1 definiert ist, mit einem geeigneten organometallischen Reagens;
d) falls n 1 oder 2 bedeutet, Oxidation des Schwefelatoms der entsprechenden Verbindung der allgemeinen Formel (I), in der n Null ist.

8. Herbizide Zusammensetzung, die als Wirkstoff eine als Herbizid wirksame Menge eines Isoxazolderivats der allgemeinen Formel (I) nach Anspruch 1 in Kombination mit einem in der Landwirtschaft akzeptablen Verdünnungsmittel oder Trägerstoff und/oder oberflächenaktiven Mittel enthält.

9. Herbizide Zusammensetzung nach Anspruch 8, die 0,05 bis 90 Gew-% des Wirkstoffs enthält.

10. Herbizide Zusammensetzung nach Anspruch 8 oder 9, die in flüssiger Form vorliegt und 0,05 bis 25 Gew.-% des oberflächenaktiven Mittels enthält.

11. Herbizide Zusammensetzung nach Anspruch 8, 9 oder 10 in Form einer als Konzentrat vorliegenden wäßrigen Suspension, eines benetzbaren Pulvers, eines wasserlöslichen oder in Wasser dispergierbaren Pulvers, eines flüssigen wasserlöslichen Konzentrats, einer als Konzentrat vorliegenden flüssigen emulgierbaren Suspension, eines Granulats oder emulgierbaren Konzentrats.

12. Verfahren zur Bekämpfung des Unkrautwachstums an einem Ort, das das Ausbringen einer herbizid wirksamen Menge eines Isoxazolderivats der allgemeinen Formel (l) nach Anspruch 1 an diesem Ort umfaßt.

13. Verfahren nach Anspruch 12, wobei der Ort ein Gebiet ist, das für den Anbau von Feldfrüchten verwendet wird oder verwendet werden soll, und die Verbindung in einer Applikationsmenge von 0,01 bis 4,0 kg/haverwendet wird.

14. Verfahren nach Anspruch 12, wobei der Ort ein Gebiet ist, das kein Anbaugebiet für Feldfrüchte ist, und die Verbindung in einer Applikationsmenge von 1,0 bis 20,0 kg/ha verwendet wird.

## Patentansprüche (Patentansprüche für folgende(n) Vertragsstaat(en) : ES)

1. Verfahren zur Herstellung eines 4-Benzoylisoxaolderivats der allgemeinen Formel 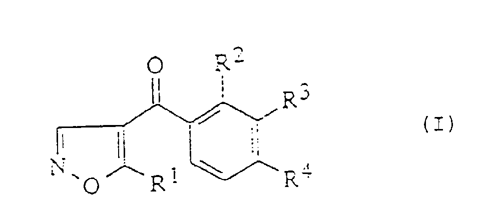 worin bedeuten:
R¹: Methyl, Ethyl, Isopropyl, Cyclopropyl oder 1-Methylcyclopropyl;
R² : eine geradkettige oder verzweigte Alkyl- oder Alkoxygruppe mit bis zu vier Kohlenstoffatomen;
R³ : ein Wasserstoffatom, Chloratom, Bromatom oder Fluoratom; oder
eine unter R⁵,-C0₂R⁵ und -OR⁵ ausgewählte Gruppe; oder
eine geradkettige oder verzweigte Alkylgruppe mit bis zu vier Kohlenstoffatomen, die mit -OR⁵ subsituiert ist; oder
eine geradkettige oder verzweigte Alkoxygruppe mit bis zu vier Kohlenstoffatomen, die mit -OR⁵ substituiert ist;
R⁴: -S(O)ₙ^{R};
R⁵: eine geradkettige oder verzweigte Alkylgruppe mit bis zu vier Kohlenstoffatomen, die ggf. mit einem oder mehreren Halogenatomen substituiert ist;
R Methyl oder Ethyl; und
n: Null, eins oder zwei;
mit der Maßgabe, daß falls R¹ Methyl oder Cyclopropyl, R² Methyl und R⁴ -S0₂Me bedeuten, R³ keine -C0₂Me oder -CO₂iPr-Gruppe ist; wobei das Verfahren umfaßt:
a) Umsetzung einer Verbindung der allgemeinen Formel (ll) : 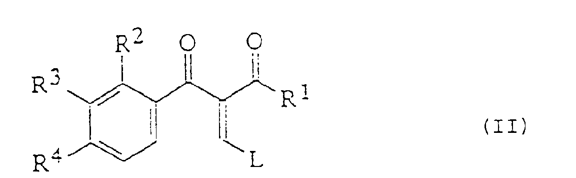 worin L eine austretende Gruppe bedeutet, und die anderen Symbole so sind, wie sie oben definiert wurden, mit einem Hydroxylaminsalz;
b) falls R⁴ eine Gruppe -SR bedeutet, Umsetzung einer Verbindung der allgemeinen Formel (lll) : 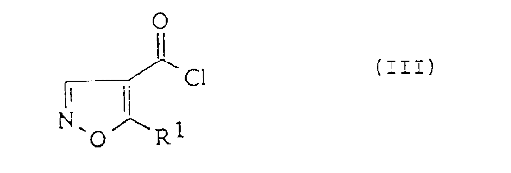 mit R¹ wie oben definiert,
mit einer Verbindung der allgemeinen Formel (IV): 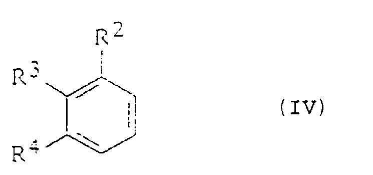 in der R⁴ eine Gruppe -SR bedeutet, und R, R² und R³ wie oben definiert vorliegen;
c) Umsetzung einer Verbindung der allgemeinen Formel (V) : 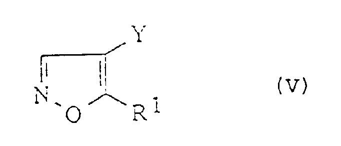 worin Y eine Carboxygruppe oder ein reaktives Derivat einer Carboxygruppe oder eine Cyanogruppe bedeutet, und R¹ wie oben definiert ist,
mit einem geeigneten organometallischen Reagens; oder
d) falls n 1 oder 2 bedeutet, Oxidation des Schwefelatoms der entsprechenden Verbindung der allgemeinen Formel (l), in der n Null ist.

2. Verfahren nach Anspruch 1, worin bedeuten:
a) R¹: Isopropyl, Cyclopropyl oder l-Methylcyclopropyl; und/oder
b) R²: Methyl, Ethyl, Methoxy oder Ethoxy; und/oder
c) R³ : ein Wasserstoffatom, Chloratom, Bromatom oder Fluoratom; oder
eine Gruppe, die unter Methyl, Methoxy, Ethoxy, -CH₂OR⁵, -O-(CH₂)₂OR⁵, und -CO₂R⁵ ausgewählt ist, worin R⁵ eine geradkettige oder verzweigte Alkylgruppe mit bis zu drei Kohlenstoffatomen bedeutet.

3. Verfahren nach Anspruch 1 oder 2, worin bedeuten:
R¹: Isopropyl, Cyclopropyl oder l-Methylcyclopropyl;
R²: Methyl, Ethyl, Methoxy oder Ethoxy, und
R³: Wasserstoff, Fluor, Chlor; Brom, Methyl, Methoxymethyl oder 1-Methoxyethoxy.

4. Verfahren nach Anspruch 1, 2 oder 3, worin bedeuten:
R¹: Isopropyl, 1-Methylcyclopropyl oder Cyclopropyl;
R²: Methyl, Ethyl, Methoxy oder Ethoxy;
R³ : Wasserstoff, Fluor, Chlor; Brom, Methyl oder Methoxy; und
R: Methyl.

5. Verfahren nach einem der vorhergehenden Ansprüche, worin bedeuten:
R¹: Cyclopropyl;
R²: Methyl oder Methoxy und R³: Methoxy oder Wasserstoff, mit der Maßgabe, daß R² und R³ nicht gleichzeitig Methoxy bedeuten;
und
R Methyl.

6. Verfahren nach Anspruch 1 für die Herstellung von:
5-Cyclopropyl-4-(2-methoxy-4-methylsulphenylbenzoyl) isoxazol;
5-Cyclopropyl-4-(2-methoxy-4-methylsulphonylbenzoyl) isoxaol;
5-Cyclopropyl-4-(2-methoxy-4-methylsulphinylbenzoyl) isoxazol;
5-Cyclopropyl-4-(2-methyl-4-methylsulphinylbenzoyl) isoxazol;
5-Cyclopropyl-4-(2-methyl-4-methylsulphonylbenzoyl) isoxazol;
5-Cyclopropyl-4-(2-methyl-4-methylsulphenylbenzoyl) isoxazol;
5-(1-Methylcyclopropyl)-4-(2-methyl-4-methylsulphonylbenzoyl)-isoxazol;
4-(2-Ethyl-4-methylsulphenylbenzoyl)-5-cyclopropylisoxazol;
4-(2-Ethyl-4-methylsulphinylbenzoyl)-5-cyclopropylisoxazol;
4-(2-Ethyl-4-methylsulphonylbenzoyl)-5-cyclopropylisoxazol;
4-(2-Ethoxy-4-methylsulphenylbenzoyl)-5-cyclopropylisoxazol;
4-(2-Ethoxy-4-methylsulphonylbenzoyl)-5-cyclopropylisoxazol;
4-(3-Chlor-2-methyl-4-methylsulphenylbenzoyl)-5-cyclopropylisoxazol;
4-(3-Chlor-2-methyl-4-methylsulphinylbenzoyl)-5-cyclopropylisoxazol;
4-(3-Chlor-2-methyl-4-methylsulphonylbenzoyl)-5-cyclopropylisoxazol;
4-(2-Methyl-3-methoxy-4-methylsulphenylbenzoyl)-5-cyclopropylisoxazol;
4-(2-Methyl-3-methoxy-4-methylsulphonylbenzoyl)-5-cyclopropylisoxazol;
4-(2,3-Dimethyl-4-methylsulphenylbenzoyl)-5-cyclopropylisoxazol;
4-(2,3-Dimethyl-4-methylsulphinylbenzoyl)-5-cyclopropylisoxazol; oder
4-(2,3-Dimethyl-4-methylsulphonylbenzoyl)-5-cyclopropylisoxazol.

7. Verfahren zur Herstellung einer herbiziden Zusammensetzung, das die Formulierung einer als Herbizid wirksamen Menge eines Isoxazolderivats der allgemeinen Formel (I) nach Anspruch 1 als Wirkstoff in Kombination mit einem in der Landwirtschaft akzeptablen Verdünnungsmittel oder Trägerstoff und/oder oberflächenaktiven Mittel umfaßt.

8. Verfahren nach Anspruch 7 zur Herstellung einer herbiziden Zusammensetzung, die 0,05 bis 90 Gew-% des Wirkstoffs enthält.

9. Verfahren nach Anspruch 7 oder 8 für die Herstellung einer herbiziden Zusammensetzung, die in flüssiger Form vorliegt und 0,05 bis 25 % oberflächenaktives Mittel enthält.

10. Verfahren nach Anspruch 9, 10 oder 11 für die Herstellung einer herbiziden Zusammensetzung in Form einer als Konzentrat vorliegenden wäßrigen Suspension, eines benetzbaren Pulvers, eines wasserlöslichen oder in Wasser dispergierbaren Pulvers, eines flüssigen wasserlöslichen Konzentrats, einer flüssigen emulgierbaren als Konzentrat vorliegenden Suspension, eines Granulats oder emulgierbaren Konzentrats.

11. Verfahren zur Bekämpfung von Unkrautwachstum an einem Ort, das das Ausbringen einer herbizid wirksamen Menge eines Isoxazolderivats der allgemeinen Formel (I) nach Anspruch 1 an diesem Ort umfaßt.

12. Verfahren nach Anspruch 11, wobei der Ort ein Gebiet ist, das für den Anbau von Feldfrüchten verwendet wird oder verwendet werden soll, und die Verbindung in einerApplikationsmenge von 0,01 bis 4,0 kg/haverwendet wird.

13. Verfahren nach Anspruch 1, wobei der Ort ein Gebiet ist, das kein Anbaugebiet für Feldfrüchte ist, und die Verbindung in einer Applikationsmenge von 1,0 bis 20,0 kg/ha verwendet wird.

## Revendications (Revendications pour l'(les) Etat(s) contractant(s) suivant(s) : AT, BE, CH, DE, DK, FR, GB, GR, IE, IT, LI, LU, NL, PT, SE)

1. Un dérivé du 4-benzoyl isoxazole répondant à la formule générale (1) : 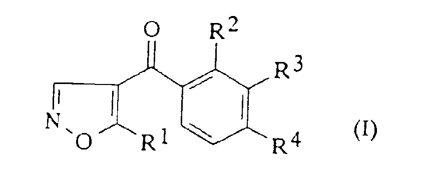 dans laquelle :
R¹ représente :
un radical méthyle, éthyle, isopropyle, cyclopropyle ou 1-méthylcydopropyle
R² représente :
un radical alkyle ou alkoxy, linéaire ou ramifié, contenant de 1 à 4 atomes de carbone ;
R³ représente :
l'hydrogène , un atome de chlore de brome ou de fluor, ou un radical choisi parmi les radicaux R⁵, -COOR^{S} et OR⁵ ;ou
un radical alkyle, linéaire ou ramifié, contenant de 1 à 4 atomes de carbone substitué par un radical OR⁵ ; ou
un radical alkyloxy, linéaire ou ramifié, contenant de 1 à 4 atomes de carbone substitué par un radical OR⁵ ; ou
R⁴ représente -S(O)ₙR
R⁵ représente un radical alkyle, linéaire ou ramifié, contenant de 1 à 4 atomes de carbone éventuellement substitué par un ou plusieurs atomes d'halogènes.
R représente un radical méthyle ou éthyle et
n représente zéro, un ou deux ;
à condition que si R¹ représente un radical méthyle ou cyclopropyle, R² représente un radical méthyle
et R⁴ représente -S0₂Me, R³ ne soit pas un radical - C0₂Me ou C0₂isoPr.

2. Un composé selon la revendication 1 dans laquelle :
a) R¹ représente :
un radical isopropyle, cyclopropyle ou 1-méthylcydopropyle ; et/ou
b) R² représente un radical méthyle, éthyle, méthoxy ou éthoxy; et/ou
c) R³ représente :
l'hydrogène, un atome chlore, de brome ou de fluor ; ou
un radical méthyle, méthoxy éthoxy, -CH₂0R⁵, -O-(CH₂)₂OR⁵, et -CO₂R⁵, dans lesquelles R⁵ représente un radical alkyle linéaire ou ramifié, contenant de 1 à 3 atomes de carbone.

3. Un composé selon la revendication 1 ou 2 dans laquelle :
R¹ représente un radical isopropyle, cyclopropyle ou 1-méthylcydopropyle ;
R² représente un radical méthyle, éthyle, méthoxy ou éthoxy ; et
R³ représente l'hydrogène, un atome chlore, de brome ou de fluor; ou un radical méthyle, méthoxyméthyle ou 1-éthoxyéthoxy.

4. Un composé selon la revendication 1, 2 ou 3 dans laquelle :
R¹ représente un radical isopropyle, cyclopropyle ou 1-méthylcydopropyle ;
R² représente un radical méthyle, éthyle, méthoxy ou éthoxy ; et
R³ représente l'hydrogène, un atome chlore, de brome ou de fluor ; ou un radical méthyle ou méthoxy
R représente un radical méthyle

5. Un composé selon l'une quelconque des revendications précédentes, dans laquelle :
R¹ représente un radical cyclopropyle;
R² représente un radical méthyle ou méthoxy; et R³ représente l'hydrogène, ou un radical méthoxy à condition que R² et R³ ne représentent pas simultanément un radical méthoxy
et R représente un radical méthyle

6. Un composé selon la revendication 1 qui est:
5-cyclopropyl-4-(2-méthoxy-4-méthylsulfènylbenzoyl)isoxazole;
5-cyclopropyl-4-(2-méthoxy-4-méthylsulfonylbenzoyl)isoxazole;
5-cyclopropyl-4-(2-méthoxy-4-méthylsulfinylbenzoyl)isoxazole;
5-cyclopropyl-4-(2-méthyl-4-méthylsulfinylbenzoyl)isoxazole;
5-cyclopropyl-4-(2-méthyl-4-méthylsulfonylbenzoyl)isoxazole;
5-cyclopropyl-4-(2-méthyl-4-méthylsulfènylbenzoyl)isoxazole;
5-(1-méthylcyclopropyl)-4-(2-méthyl-4-méthylsulfonylbenzoyl)isoxazole;
4-(2-éthyl-4-méthylsulfènylbenzoyl)-5-cyclopropylisoxazole;
4-(2-éthyl-4-méthylsulfinylbenzoyl)-5-cyclopropylisoxazole;
4-(2-éthyl-4-méthylsulfonylbenzoyl)-5-cyclopropylisoxazole;
4-(2-éthoxy-4-méthylsulfènylbenzoyl)-5-cyclopropylisoxazole;
4-(2-éthoxy-4-méthylsulfonylbenzoyl)-5-cyclopropylisoxazole;
4-(3-chloro-2-méthyl-4-méthylsulfénylbenzoyl)-5-cyclopropylisoxazole;
4-(3-chloro-2-méthyl-4-méthylsulfinylbenzoyl)-5-cyclopropylisoxazole;
4-(3-chloro-2-méthyl-4-méthylsulfonylbenzoyl)-5-cyclopropylisoxazole;
4-(2-méthyl-3-méthoxy-4-méthylsulfènylbenzoyl)-5-cyclopropylisoxazole ;
4-(2-méthyl-3-méthoxy-4-méthylsulfonylbenzoyl)-5-cyclopropylisoxazole;
4-(2,3-diméthyl-4-méthylsulfènylbenzoyl)-5-cyclopropylisoxazole;
4-(2,3-diméthyl-4-méthylsulfinylbenzoyl)-5-cyclopropylisoxazole;
4-(2,3-diméthyl-4-méthylsulfonylbenzoyl)-5-cyclopropylisoxazole.

7. Un procédé de préparation d'un composé de formule générale (1) selon la revendication 1 qui comprend :
a) la réaction d'un composé de formule générale (ll) : 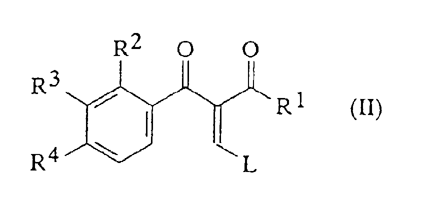 dans laquelle L est un groupe partant, avec un sel d'hydroxylamine.
b) si R⁴ représente un radical -SR, la réaction d'un composé de formule générale (lll) 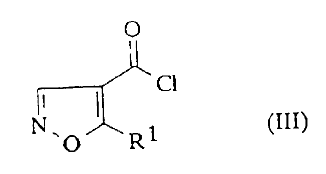 dans lequel R¹ a la définition donnée dans la revendication 1 avec un composé de formule générale (IV) : 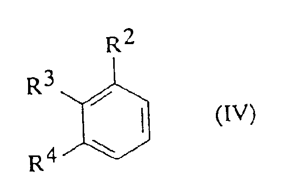 dans laquelle R⁴ représente un radical -SR et R, R² et R³ sont définis comme dans la revendication 1 ;
c) la réaction d'un composé de formule générale (V) : 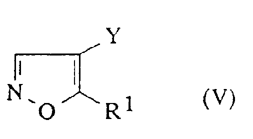 dans laquelle Y représente un radical carboxy ou un dérivé réactif d'un radical carboxy, avec un réactif organométallique approprié ;
d) lorsque n représente 1 ou 2, l'oxydation de l'atome de soufre du composé correspondant de formule générale (1) dans lequel n est égal à 0.

8. Une composition herbicide qui contient comme matière active une quantité herbicide efficace d'un dérivé de l'isoxazole de formule générale (1) tel que défini dans la revendication 1) en association avec un diluant ou une charge et/ou un agent tensioactif acceptable pour des applications agricoles

9. Une composition herbicide selon la revendication 8 qui contient de 0,05 à 90% en poids de la matière active.

10. Une composition herbicide selon l'une quelconque des revendications 8 ou 9 qui est sous forme liquide et contient de 0,05 à 25% en poids d'un agent tensioactif..

11. Une composition herbicide selon l'une quelconque des revendications 8, 9 ou 10 qui se présente sous la forme d'une suspension aqueuse concentrée, d'une poudre mouillable, d'un concentré liquide soluble dans l'eau, d'une suspension concentrée liquide émulsionnable, d'un granulé ou d'un concentré émulsionnable.

12. Un procédé de contrôle de la croissance des mauvaises herbes en un lieu qui consiste à appliquer sur le dit lieu une quantité herbicide efficace d'un dérivé de l'isoxazole de formule générale (1) tel que défini dans la revendication 1.

13. Un procédé selon la revendication 12 dans lequel le lieu est une zone utilisée, ou destinée à être utilisée pour y faire pousser des cultures et ou la dose d'application du composé est comprise entre 0,01 et 4,0 kg par hectare.

14. Un procédé selon la revendication 12 dans lequel le lieu est une zone de non culture et ou la dose d'application du composé est comprise entre 1 et 20,0 kg par hectare.

## Revendications (Revendications pour l'(les) Etat(s) contractant(s) suivant(s) : ES)

1. Un procédé de préparation d'un dérivé du 4-benzoyl isoxazole de formule générale 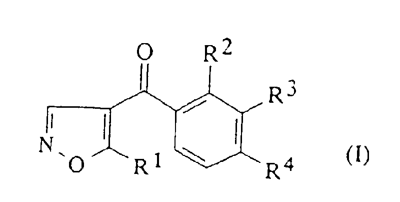 dans laquelle :
R¹ représente :
un radical méthyle, éthyle, isopropyle, cyclopropyle ou 1-méthylcydopropyle;
R² représente :
un radical alkyle ou alkoxy, linéaire ou ramifié, contenant de 1 à 4 atomes de carbone ;
R³ représente :
l'hydrogène , un atome de chlore de brome ou de fluor, ou un radical choisi parmi les radicaux R⁵, -COOR^{S} et OR⁵ ;ou
un radical alkyle, linéaire ou ramifié, contenant de 1 à 4 atomes de carbone substitué par un radical OR⁵ ; ou
un radical alkyloxy, linéaire ou ramifié, contenant de 1 à 4 atomes de carbone substitué par un radical OR⁵ ; ou
R⁴ représente -S(O)ₙR
R⁵ représente un radical alkyle, linéaire ou ramifié, contenant de 1 à 4 atomes de carbone éventuellement substitué par un ou plusieurs atomes d'halogènes.
R représente un radical méthyle ou éthyle et
n représente zéro, un ou deux ;
à condition que si R¹ représente un radical méthyle ou cyclopropyle, R² représente un radical méthyle et R⁴ représente -SO₂Me, R³ ne soit pas un radical -CO₂Me ou C0₂isoPr
le dit procédé comprenant les réactions suivantes :
a) la réaction d'un composé de formule générale (ll) : 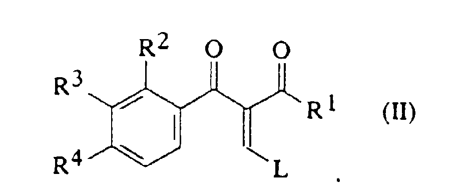 dans laquelle L est un groupe partant, avec un sel d'hydroxylamine.
b) si R⁴ représente un radical -SR, la réaction d'un composé de formule générale (lll) 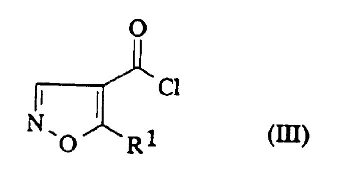 dans lequel R¹ a la définition donnée ci-dessus avec un composé de formule générale (IV) : 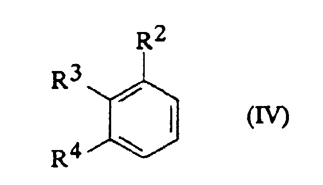 dans laquelle R⁴ représente un radical -SR et R, R² et R³ sont définis comme ci-dessus;
c) la réaction d'un composé de formule générale (V) : 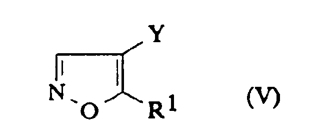 dans laquelle Y représente un radical carboxy ou un dérivé réactif d'un radical carboxy, ou un radical cyano et R¹ a la définition donnée ci-dessus, avec un réactif organométallique approprié ; ou
d) lorsque n représente 1 ou 2, l'oxydation de l'atome de soufre du composé correspondant de formule générale (l) dans lequel n est égal à 0.

2. Un procédé selon la revendication 1 dans lequel :
a) R⁷ représente :
un radical isopropyle, cyclopropyle ou 1-méthylcydopropyle ; et/ou
b) R² représente un radical méthyle, éthyle, méthoxy ou éthoxy ; et/ou
c) R³ représente :
l'hydrogène, un atome chlore, de brome ou de fluor ; ou
un radical méthyle, méthoxy, éthoxy, -CH₂0R⁵, -O-(CH₂)₂OR⁵, et -C0₂R⁵, dans lesquelles R⁵ représente un radical alkyle linéaire ou ramifié, contenant de 1 à 3 atomes de carbone.

3. Un procédé selon la revendication 1 ou 2 dans laquelle :
R¹ représente un radical isopropyle, cyclopropyle ou 1-méthylcyclopropyle;
R² représente un radical méthyle, éthyle, méthoxy ou éthoxy ; et
R³ représente l'hydrogène, un atome chlore, de brome ou de fluor; ou un radical méthyle, méthoxyméthyle ou 1-méthoxyéthoxy.

4. Un procédé selon la revendication 1, 2 ou 3 dans laquelle :
R¹ représente un radical isopropyle, cyclopropyle ou 1-méthylcyclopropyle ;
R² représente un radical méthyle, éthyle, méthoxy ou éthoxy;
R³ représente l'hydrogène, un atome chlore, de brome ou de fluor ; ou un radical méthyle ou méthoxy ; et R représente un radical méthyle

5. Un procédé selon l'une quelconque des revendications précédentes, dans laquelle :
R¹ représente un radical cyclopropyle;
R² représente un radical méthyle ou méthoxy; et R³ représente l'hydrogène, ou un radical méthoxy à condition que R² et R³ ne représentent pas simultanément un radical méthoxy.
et R représente un radical méthyle

6. Un procédé selon la revendication 1 pour la préparation du :
5-cyclopropyl-4-(2-méthoxy-4-méthylsulfènylbenzoyl)isoxazole;
5-cyclopropyl-4-(2-méthoxy-4-méthylsulfonylbenzoyl)isoxazole;
5-cyclopropyl-4-(2-méthoxy-4-méthylsulfinylbenzoyl)isoxazole;
5-cyclopropyl-4-(2-méthyl-4-méthylsulfinylbenzoyl)isoxazole;
5-cyclopropyl-4-(2-méthyl-4-méthylsulfonylbenzoyl)isoxazole;
5-cyclopropyl-4-(2-méthyl-4-méthylsulfènylbenzoyl)isoxazole;
5-(1-méthylcyclopropyl)-4-(2-méthyl-4-méthylsulfonylbenzoyl)isoxazole;
4-(2-éthyl-4-méthylsulfènylbenzoyl)-5-cydopropylisoxazole;
4-(2-éthyl-4-méthylsulfinylbenzoyl)-5-cyclopropylisoxazole;
4-(2-éthyl-4-méthylsulfonylbenzoyl)-5-cydopropylisoxazole;
4-(2-éthoxy-4-méthylsulfènylbenzoyl)-5-cyclopropylisoxazole;
4-(2-éthoxy-4-méthylsulfonylbenzoyl)-5-cyclopropylisoxazole;
4-(3-chloro-2-méthyl-4-méthylsulfènylbenzoyl)-5-cyclopropylisoxazole;
4-(3-chloro-2-méthyl-4-méthylsulfinylbenzoyl)-5-cyclopropylisoxazole;
4-(3-chloro-2-méthyl-4-méthylsulfonylbenzoyl)-5-cyclopropylisoxazole;
4-(2-méthyl-3-méthoxy-4-méthylsulfènylbenzoyl)-5-cyclopropylisoxazole;
4-(2-méthyl-3-méthoxy-4-méthylsulfonylbenzoyl)-5-cyclopropylisoxazole;
4-(2,3-diméthyl-4-méthylsulfènylbenzoyl)-5-cyclopropylisoxazole;
4-(2,3-diméthyl-4-méthylsυlfinylbenzoyl)-5-cyclopropylisoxazole;
4-(2,3-diméthyl-4-méthylsulfonylbenzoyl)-5-cyclopropylisoxazole.

7. Un procédé de préparation d'une composition herbicide qui comprend la formulation comme matière active d'une quantité herbicide efficace d'un dérivé de l'isoxazole de formule générale (1) tel que défini dans la revendication 1 en association avec un diluant ou une charge et/ou un agent tensioactif.

8. Un procédé selon la revendication 7 pour la préparation d'une composition herbicide qui contient de 0,05 à 90% en poids de matière active.

9. Un procédé selon la revendication 7 ou 8 pour la préparation d'une composition herbicide sous forme liquide contenant de 0,05 à 25% en poids d'un agent tensioactif.

10. Un procédé selon la revendication 9, 10 ou 11 pour la préparation d'une composition herbicide sous forme d'une suspension aqueuse concentrée, d'une poudre mouillable ou d'une poudre dispersable dans l'eau, d'un concentré liquide soluble dans l'eau, d'une suspension concentrée liquide émulsionnable, d'un granulé ou d'un concentré émulsionnable.

11. Un procédé de contrôle de la croissance des mauvaises herbes en un lieu qui consiste à appliquer sur le dit lieu une quantité herbicide efficace d'un dérivé de l'isoxazole de formule générale (1 ) tel que défini dans la revendication 1.

12. Un procédé selon la revendication 11 dans lequel le lieu est une zone utilisée, ou destinée à être utilisée pour y faire pousser des cultures et ou la dose d'application du composé est comprise entre 0,01 et 4,0 kg par hectare.

13. Un procédé selon la revendication 11 dans lequel le lieu est une zone de non culture et ou la dose d'application du composé est comprise entre 1 et 20,0 kg par hectare.
